(19) Europäisches Patentamt / European Patent Office / Office européen des brevets

(11) **EP 3 478 449 B1**

(12) **FASCICULE DE BREVET EUROPEEN**

(45) Date de publication et mention
de la délivrance du brevet:
**08.09.2021 Bulletin 2021/36**

(21) Numéro de dépôt: **17745419.6**

(22) Date de dépôt: **03.07.2017**

(51) Int Cl.:
*A61B 17/00* (2006.01)     *A61B 90/00* (2016.01)
*B25B 9/02* (2006.01)     *A61B 17/30* (2006.01)

(86) Numéro de dépôt international:
**PCT/FR2017/051790**

(87) Numéro de publication internationale:
**WO 2018/007733 (11.01.2018 Gazette 2018/02)**

(54) **PINCE MECATRONIQUE**

MECHATRONISCHE ZANGE

MECHATRONIC FORCEPS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.07.2016 FR 1656359**

(43) Date de publication de la demande:
**08.05.2019 Bulletin 2019/19**

(73) Titulaires:
• **Percipio Robotics**
**25000 Besançon (FR)**
• **SORBONNE UNIVERSITE**
**75006 Paris (FR)**
• **Centre National de la Recherche Scientifique
(CNRS)**
**75016 Paris (FR)**

(72) Inventeurs:
• **REGNIER, Stéphane**
**92270 BOIS COLOMBES (FR)**

• **DAUNIZEAU, Thomas**
**75015 PARIS (FR)**
• **HALIYO, Dogan Sinan**
**75011 PARIS (FR)**
• **LU, Tianming**
**94200 IVRY SUR SEINE (FR)**
• **LUTRINGER, Marie**
**25870 GENEUILLE (FR)**
• **HERIBAN, David**
**25480 ECOLE VALENTIN (FR)**

(74) Mandataire: **Cabinet Netter**
**36, avenue Hoche**
**75008 Paris (FR)**

(56) Documents cités:
EP-A1- 1 334 700     EP-A1- 1 584 414
EP-A1- 1 627 707     CH-A5- 685 749
DE-B4- 19 782 304

EP 3 478 449 B1

**Description**

La présente invention se rapporte à une pince mécatronique.

La manipulation d'objets macroscopique et microscopiques trouve aujourd'hui son application dans de nombreux domaines technologiques, dont notamment l'électronique, le médical et l'industrie horlogère.

La miniaturisation des objets requiert une évolution des dispositifs de manipulation, tels que les pinces de micromanipulation ou les pinces robotiques. En effet, il existe un besoin pour les pinces de pouvoir réaliser des manipulations de plus en plus précises.

Il existe des pinces comprenant des mécanismes d'assistance à la manipulation pour les opérateurs. Il s'agit classiquement de systèmes motorisés pour ouvrir ou fermer les branches d'une pince. Les pinces comportent généralement un élément de contrôle de type manette bâtonnet (en anglais : *Joystick*).

Le document WO 2015/158945 divulgue une pince comprenant des branches entraînées par un moteur. Les branches sont agencées parallèlement l'une par rapport à l'autre. Cet agencement est conservé lors de l'ouverture et de la fermeture des branches.

Lorsque les manipulations concernent des objets à l'échelle micrométrique, les opérations manuelles par des opérateurs sont impossibles. Il est fait appel à un système robotique pour piloter ce type de manipulations. Toutefois, l'intervention de systèmes robotiques requiert des interfaces homme-machine. Ces interfaces sont rarement intuitives et rendent difficile le pilotage de la pince. Par ailleurs, les phénomènes physiques rencontrés à l'échelle micrométrique (effets surfaciques d'adhésions etc.) ne sont que très difficilement contrôlables voir non-contrôlables.

De plus, les manipulations d'objets à l'échelle submillimétrique par un opérateur sont difficiles, voire impossibles, avec les solutions de l'état de la technique. Les dispositifs de l'état de la technique sont généralement encombrants. Les contraintes de dimensionnement ne sont pas satisfaisant et ne répondent pas aux besoins des opérateurs. Par ailleurs, les dispositifs de l'état de la technique ne sont pas satisfaisant en termes énergétiques.

Le document EP1584414 divulgue une pince électrique comprenant un élément de préhension pour libérer / saisir un objet, un élément de base ayant une partie d'engagement à engager avec l'autre partie d'extrémité de l'élément de préhension, un élément de support pour coupler de manière fixe une partie d'extrémité de la préhension élément et ayant une partie cylindrique creuse pénétrant, et un interrupteur pour faire tourner et arrêter un moteur.

La présente invention vient améliorer la situation.

À cet effet, l'invention vient introduire un dispositif mécatronique comprenant deux branches s'étendant généralement à partir d'une embase dans une première direction et conformées pour être prises conjointement en main par un opérateur, les deux branches comportant des extrémités libres formant pince et agencées pour se rapprocher par rotation l'une de l'autre depuis une position de repos à une position active, un moteur logé dans l'embase, un actionneur monté entre les branches, et un moyen de commande dudit actionneur, l'actionneur étant lié au moteur de façon à être entraîné en un mouvement de translation selon la première direction, l'actionneur possédant au moins une première surface de contact agencée pour former came avec au moins une deuxième surface de contact respective située sur chaque branche, la ou les premières surfaces de contact demeurant en prise avec la ou les deuxièmes surfaces de contact respectives, le moyen de commande de l'actionneur étant agencé pour contrôler ledit mouvement de translation de l'actionneur afin de produire un mouvement de rotation desdites branches qui exerce une force choisie de la pince dans sa position active tel que défini dans la revendication 1.

Il s'agit d'une pince mécatronique portable et active. La pince mécatronique est agencée de telle façon que l'effort de préhension perçu par l'opérateur peut être découplé de l'effort résultant de la saisie d'un objet.

Selon un mode de réalisation la force choisie peut être une force à l'encontre du rapprochement par rotation desdites extrémités libres. Il s'agit notamment d'une résistance ressentie par l'opérateur lors de la saisie d'un objet. Selon un autre mode de réalisation, force choisie peut être une force en assistance du rapprochement par rotation desdites extrémités libres. Il s'agit notamment d'une aide ressenti par l'opérateur lors de la saisie d'un objet.

Ainsi, la pince mécatronique de l'invention offre plusieurs modes opérationnels et avantages lors de son utilisation par un opérateur. Tout particulièrement, la pince mécatronique permet de ressentir un effort s'opposant à la fermeture de la pince sans qu'aucun objet physique ne soit manipulé. La pince mécatronique permet également de saisir un objet sans avoir à exercer un effort de fermeture avec les doigts. À ces exemples de fonctionnements extrémaux s'ajoutent l'ensemble des fonctionnements intermédiaires dans lesquelles les efforts de saisie et de préhension sont non nuls (mais néanmoins toujours décorrélés). Par ailleurs, la pince mécatronique permet de simuler la saisie d'un objet sans qu'un objet physique soit effectivement saisi (saisie virtuelle d'objet). La pince mécatronique peut aussi être utilisée de façon automatique et/ou robotique. Incidemment, la pince mécatronique peut également être employée en tant que pince traditionnelle lorsque le système actif de l'invention est désactivé (à savoir lorsque la force choisie est nulle).

En pratique, l'effort perçu par l'opérateur, à savoir l'effort qui résulte de la prise en main et du maintien de la pince, est rendu indépendant de l'effort provenant de la saisie d'un objet grâce au mécanisme actif de la pince mécatronique.

Avantageusement, l'actionneur de l'invention est disposé sur un axe longitudinal de la pince mécatronique.

Avantageusement, il s'agit de l'axe longitudinal central de la pince mécatronique. Ceci permet de minimiser les efforts nécessaires pour agir sur la rotation des branches.

Le mouvement de translation de l'actionneur selon la première direction peut être choisi parmi un déplacement de l'actionneur sur l'axe longitudinal dans le premier sens de cet axe et un déplacement de l'actionneur sur l'axe longitudinal dans le deuxième sens de cet axe. En pratique, il s'agit d'une poussée ou d'une traction de l'actionneur permettant respectivement d'ouvrir et de fermer la pince par rotation des branches. Un avantage des déplacements sensiblement le long de l'axe longitudinal est notamment la symétrie de mouvement de rotation des branches ainsi que la minimisation des forces nécessaires pour agir sur la rotation.

L'actionneur peut comprendre une vis-à-billes. Un avantage de l'utilisation d'une vis-à-billes est notamment sa bidirectionnalité. De plus, la vis-à-billes utilisée dans l'invention présente l'avantage de frottements très faibles. À cela s'ajoute que l'angle d'hélice est grand, ce qui offre

L'actionneur peut comprendre une vis-à-billes. Un avantage de l'utilisation d'une vis-à-billes est notamment sa bidirectionnalité. De plus, la vis-à-billes utilisée dans l'invention présente l'avantage de frottements très faibles. À cela s'ajoute que l'angle d'hélice est grand, ce qui offre un haut rendement et par conséquent une bonne réversibilité. Enfin, la vis-à-billes possède un grand rapport diamètre sur longueur, ce qui assure une bonne rigidité. Cela est favorable en regard de la bande passante, c'est-à-dire en regard de la fréquence maximale que peut avoir la force choisie. La bande passante dépend sensiblement de la nature de l'actionneur ainsi que de la rigidité et de la masse des constituants de la chaîne cinématique. Plus ces éléments sont rigides et légers, plus la bande passante est grande. Cela présente notamment un intérêt pour une application en vue de la création de signaux tactiles. Les doigts humains sont des capteurs performants capables de capter/mesurer des fréquences allant jusqu'à 500 Hz. En vue de couvrir l'intégralité de ce spectre haptique, la pince mécatronique prévoit d'exciter les doigts de l'opérateur jusqu'à cette fréquence.

L'actionneur est lié au moteur au moyen d'un organe de couplage flexible. Un avantage de l'invention est notamment la souplesse de construction. En effet, l'organe de couplage flexible permet de palier des écarts potentiels par rapport à l'axe longitudinal lors de l'assemblage de la pince mécatronique.

Dans un mode de réalisation, l'actionneur comprend un ou plusieurs galets montés en rotation, chaque galet formant la première surface de contact. Dans ce mode de réalisation, chaque branche comprend un ou plusieurs épaulements faisant saillie vers la branche opposée, chaque épaulement formant la deuxième surface de contact respective.

Dans un autre mode de réalisation, l'actionneur comprend un ou plusieurs plans inclinés, chaque plan incliné formant ladite première surface de contact. Dans ce mode de réalisation, chaque branche comprend un ou plusieurs galets montés en rotation, chaque galet formant la deuxième surface de contact respective.

Dans l'un ou l'autre mode de réalisation précité, respectivement comprenant un ensemble galet(s)/épaulement(s) et un ensemble plan(s) incliné(s)/galet(s), il est formé une came afin de transformer le mouvement de translation de l'actionneur en un mouvement de rotation des branches de la pince mécatronique. Un avantage de cet agencement est que la came de l'invention présente de très faibles contraintes de frottements.

Il faut noter la synergie existante entre plusieurs éléments constituants de la pince mécatronique en termes de minimisation de frottements. Ceci présente plusieurs avantages et notamment en termes de manipulation, d'ergonomie, ainsi qu'en termes d'économie d'énergie.

Le dispositif mécatronique de l'invention est agencé de sorte que la ou les premières surfaces de contact et la ou les deuxièmes surfaces de contact respectives demeurent en prise. À cet effet, la pince mécatronique de l'invention peut comprendre un premier composant magnétique à proximité de la ou des premières surfaces de contact, et un deuxième composant magnétique à proximité de la ou des deuxièmes surfaces de contact. Les premier et deuxième composants magnétiques s'attirent mutuellement de façon à assurer au moins partiellement la situation de prise entre la ou les premières surfaces de contact et la ou les deuxièmes surfaces de contact respectives.

Dans ce mode de réalisation, chaque ensemble formant came comporte donc un champ magnétique dont l'effort plaque le(s) galet(s) sur le(s) épaulement(s) ou plan(s) incliné(s). Ceci a notamment pour avantage de garantir la transmission de mouvement de l'actionneur vers les branches et, inversement le cas échéant. Les efforts de poussée et de traction sur l'actionneur sont transmis aux branches pour engager une rotation de celles-ci.

Les premier et deuxième composants magnétiques peuvent comprendre indépendamment l'un de l'autre un aimant permanent, un corps ferromagnétique et/ou un électro-aimant. Avantageusement, un circuit magnétique peut être construit autour des cames au moyen d'un ou plusieurs aimants permanent et des noyaux/récepteurs ferromagnétiques. Cet agencement résulte notamment en un mécanisme de transmission permettant généralement d'éviter tout écart ou décalage entre la/les première(s) surfaces de contact et la/les deuxième(s) surfaces de contact respectives.

Dans un mode de réalisation, la force choisie de la pince mécatronique de l'invention peut avoir une valeur comprise entre -50 N et 50 N, de préférence entre -30 N et 30 N. La mesure de la force est déduite par une mesure du courant traversant le bobinage du moteur. En tout état de cause, en utilisation active la force choisie est toujours non nulle ($\neq$ 0 N).

Dans un mode de réalisation, le moyen de commande du dispositif mécatronique de l'invention comprend une base de données de forces prédéfinies enregistrée sur une mémoire de stockage. Dans ce mode de réalisation, la force choisie peut être sélectionnée parmi ces forces prédéfinies. La base de données peut être évolutive et comprendre des données mesurées et récoltées lors de l'utilisation de la pince. Un avantage est notamment la simulation de forces engagées lors de la manipulation d'objets virtuelles avec la pince mécatronique.

Dans un mode de réalisation les extrémités des branches peuvent comprendre des embouts interchangeables. Ceci offre de nombreux avantages tels que l'utilisation d'embouts spécifiquement adaptés pour des objets donnés ou le remplacement des embouts lorsqu'ils cassent.

Dans un mode de réalisation, le dispositif mécatronique de l'invention peut comprendre un ou plusieurs capteurs. Le ou les capteurs peuvent être des capteurs de forces pour mesurer les forces appliqués en utilisation de la pince ou encore des capteurs de vitesse, des capteurs de position, des capteurs de température, des capteurs d'accélération ou des capteurs magnétiques. L'avantage est notamment la mesure des données en temps réel lors de l'utilisation du dispositif de l'invention. Ceci permet notamment de contrôler et d'agir en temps réel sur la force choisie avec le moyen de commande. En outre, les capteurs permettent de constituer la base de données relative à l'utilisation de la pince de l'invention.

D'autres avantages et caractéristiques de l'invention apparaîtront à la lecture de la description détaillée ci-après et sur les dessins annexés sur lesquels :

- la figure 1 montre un schéma fonctionnel d'un dispositif mécatronique selon l'invention ;
- la figure 2 montre un organigramme d'un procédé de commande du dispositif mécatronique de l'invention ;
- la figure 3 montre une vue en perspective d'un dispositif mécatronique selon un mode de réalisation de l'invention ;
- la figure 4 montre une vue en perspective d'une coupe du dispositif de la figure 3 ;
- la figure 5 montre une vue de dessus du dispositif de la figure 3 ;
- la figure 6 montre une vue rapprochée en perspective d'une première coupe partielle du dispositif de la figure 3 ;
- la figure 7 montre une vue rapprochée en perspective d'une deuxième coupe partielle du dispositif de la figure 3 ; et
- la figure 8 montre une photographie d'un dispositif mécatronique pris en main d'un opérateur.

Les dessins et la description ci-après contiennent, pour l'essentiel, des éléments de caractère certain. Ils font partie intégrante de la description, et pourront donc non seulement servir à mieux faire comprendre la présente invention, mais aussi contribuer à sa définition, le cas échéant.

Pour la mise au point d'une pince capable de surmonter les problèmes de l'état de la technique, la Demanderesse s'est orientée vers la mécatronique. La mécatronique est un domaine pluridisciplinaire qui combine des techniques de génie mécanique, de l'électronique, de l'automatisme et de l'informatique.

La norme NF E 01-010 propose la définition suivante pour la mécatronique : *démarche visant l'intégration en synergie de la mécanique, l'électronique, l'automatique et l'informatique dans la conception et la fabrication d'un produit en vue d'augmenter et/ou d'optimiser sa fonctionnalité.*

La Demanderesse a développé une pince capable de transmettre à un opérateur une force de préhension décorrélée de l'effort de réaction de l'objet manipulé. Réciproquement, la pince est apte à saisir un objet avec un effort indépendant de celui exercé par les doigts de l'opérateur. La pince de l'invention garantie par ailleurs une utilisation naturelle. Les contraintes sur la conception mécanique ont été surmontées. La pince de l'invention présente une excellente réversibilité, forte compacité, faible masse et faible consommation électrique par rapport aux solutions de l'état de la technique.

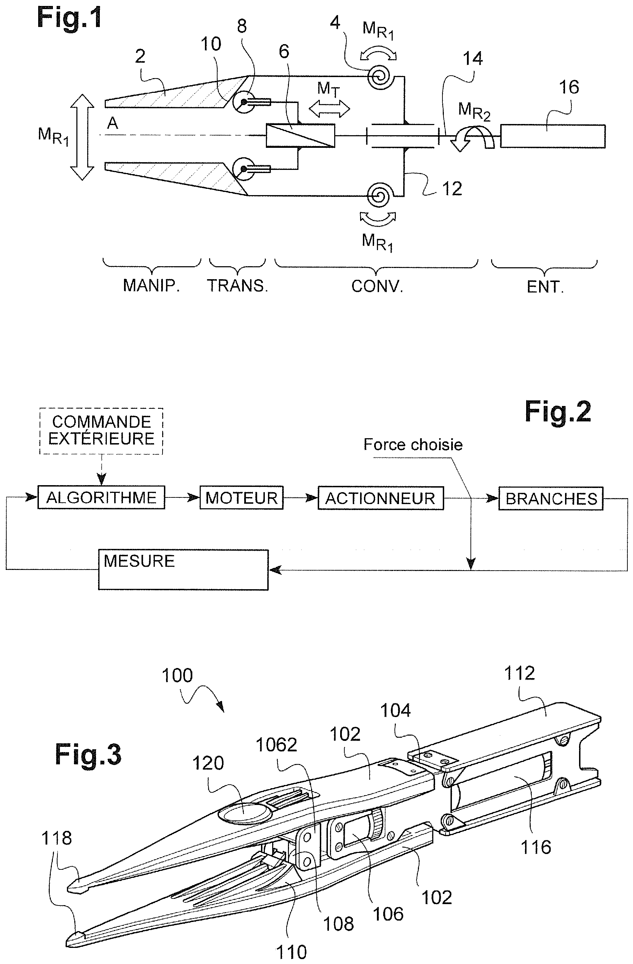

La figure 1 montre un schéma fonctionnel d'un dispositif mécatronique selon l'invention. Le dispositif présente un aspect général en forme de pince brucelles droite. Ainsi, le dispositif présente une symétrie générale par rapport à un axe longitudinal central A. L'architecture mécanique est symétrique.

Deux branches 2 sont respectivement agencées de part et d'autre de l'axe A. Les branches 2 sont montées de façon à être aptes de s'approcher l'une de l'autre par un mouvement de rotation $M_{R1}$. À cet effet, chaque branche est fixée sur un bâti 12 par un point de rotation 4 (ou point de pivot). Les extrémités libres des branches peuvent ainsi s'approcher mutuellement pour former une pince.

La rotation $M_{R1}$ des branches 2 est réversible. Le rapprochement des branches 2 est réalisé à partir d'une position de repos vers une position active. La position de repos correspond à une situation dans laquelle aucune contrainte n'est appliquée aux branches 2. Tout écart de la position de repos est une position active. Par exemple, toute situation rapprochée des branches 2 est une position active. En position de repos, aucune contrainte active n'est exercée sur le point de rotation 4. En position active, une contrainte de force variable est exercée sur le point de rotation 4. L'agencement du dispositif mécatronique permet une prise en main similaire à la prise en main d'une pince brucelles classique.

Le dispositif mécatronique comprend un moteur 16. Le moteur 16 est agencé pour entrainer un actionneur 6. Optionnellement, un organe de couplage 14 est disposé entre le moteur 16 et l'actionneur 6. L'organe de couplage 14 est de préférence flexible (aussi appelé accouplement souple) de manière à pouvoir compenser d'éventuels écarts de l'axe A entre le moteur 16 et l'actionneur 6.

Le moteur 16 est agencé pour engager un mouvement de rotation $M_{R2}$ générant un mouvement de translation $M_T$ au niveau de l'actionneur. Pour cela, l'actionneur 6 est agencé pour transformer le mouvement de rotation $M_{R2}$ engagé par le moteur 16 en mouvement de translation $M_T$. Des actionneurs comprenant des systèmes de type vis/écrou permettent notamment de transformer un mouvement de rotation (vis) et un mouvement de translation (écrou).

Dans un autre mode de fonctionnement, le moteur 16 peut engager directement un mouvement de translation au niveau de l'actionneur, et ce sans nécessiter une quelconque transformation de mouvement(s).

L'actionneur 6 comprend au moins une première surface de contact 8. Chaque branche 2 comprend au moins une deuxième surface de contact 10. Chaque première surface de contact 8 est au contact d'une deuxième surface de contact 10 respective. L'ensemble de la ou des première(s) surface(s) de contact 8 et la ou des deuxième(s) surface(s) de contact 10 forme(nt) une came. La came permet de transformer le mouvement de translation $M_T$ de l'actionneur 6 en un mouvement de rotation $M_{R1}$ des branches.

Chaque première surface 8 de contact et chaque deuxième surface de contact 10 respective demeurent constamment en prise. Ainsi, une traction de l'actionneur 6 (sur l'axe A dans un sens dirigé vers le bâti 12 sur la figure 1) a pour conséquence le rapprochement des deux branches l'une de l'autre. À l'inverse une poussée de l'actionneur 6 (sur l'axe A dans un sens dirigé vers les extrémités des branches 2 sur la figure 1) a pour conséquence l'écartement des deux branches l'une de l'autre.

D'une manière générale, le dispositif mécatronique présente quatre parties fonctionnelles :

- une partie d'entraînement ENT. comprenant notamment la ou les éléments pouvant engager une force d'entraînement, tel que le moteur 16 et les éléments associés comme un arbre moteur et une alimentation (batterie par exemple) ;
- une partie de conversion CONV. comprenant notamment la ou les éléments permettant de transformer l'entraînement du moteur en un mouvement de translation de l'actionneur 6, tel qu'une pièce de l'actionneur 6 de type vis/écrou ;
- une partie de transmission TRANS. comprenant notamment la ou les pièces permettant d'engager un mouvement des branches à partir de la translation de l'actionneur 6, tel que la première surface de contact 8 et la deuxième surface de contact 10 ; et
- une partie de manipulation MANIP. comprenant notamment la ou les pièces capables d'appréhender un objet, tel que les extrémités des branches 2 formant pince.

La figure 2 montre un organigramme d'un procédé de commande du dispositif mécatronique de l'invention. Une opération COMMANDE EXTÉRIEURE comprend la commande par un opérateur ou par un système robotique de paramétrages d'entraînements d'un moteur selon des critères variables. Une opération ALGORITHME transmet les paramètres d'entraînement à un moteur. L'opération ALGORITHME comprend notamment des calculs de conversion pour générer puis transmettre au moteur des paramètres ajustés selon les choix fixés lors de l'opération COMMANDE EXTÉRIEURE. Une opération MOTEUR comprend le fonctionnement actif d'un moteur selon le ou les paramètres choisis lors de l'opération COMMANDE EXTÉRIEURE et, le cas échéants, ajustés lors de l'opération ALGORITHME. Le fonctionnement actif du moteur dans l'opération MOTEUR a pour conséquence l'entrainement d'un actionneur dans un mouvement de translation dans une opération ACTIONNEUR. L'entrainement de l'actionneur agit directement sur des branches du dispositif mécatronique en engagent un mouvement de rotation de celles-ci dans l'opération BRANCHES. La force choisie appliquée par l'actionneur sur les branches est définie par l'ensemble des opérations COMMANDE EXTÉRIEURE et ALGORITHME. De cette manière l'opérateur ou le système robotique peuvent commander le dispositif mécatronique. Les forces résultantes de l'entraînement de l'actionneur, son positionnement et/ou son action sur les branches peuvent être mesurées au moyen de capteurs durant l'opération ACTIONNEUR. Similairement, les forces résultantes du mouvement des deux branches, leurs positionnements respectifs et/ou leur action sur un objet peuvent être mesurés au moyen de capteurs durant l'opération BRANCHES. Les opérations ACTIONNEUR et BRANCHES sont ainsi contrôlées activement par l'opérateur ou le système robotique dans une opération MESURE. Les résultats des mesures effectuées par les capteurs lors de l'opération MESURE de contrôle actif sont intégrés dans les calculs de l'opération ALGORITHME afin de contrôler la force choisie appliquée au dispositif mécatronique et ainsi de décorréler l'effort de l'opérateur par rapport à une prise d'objet.

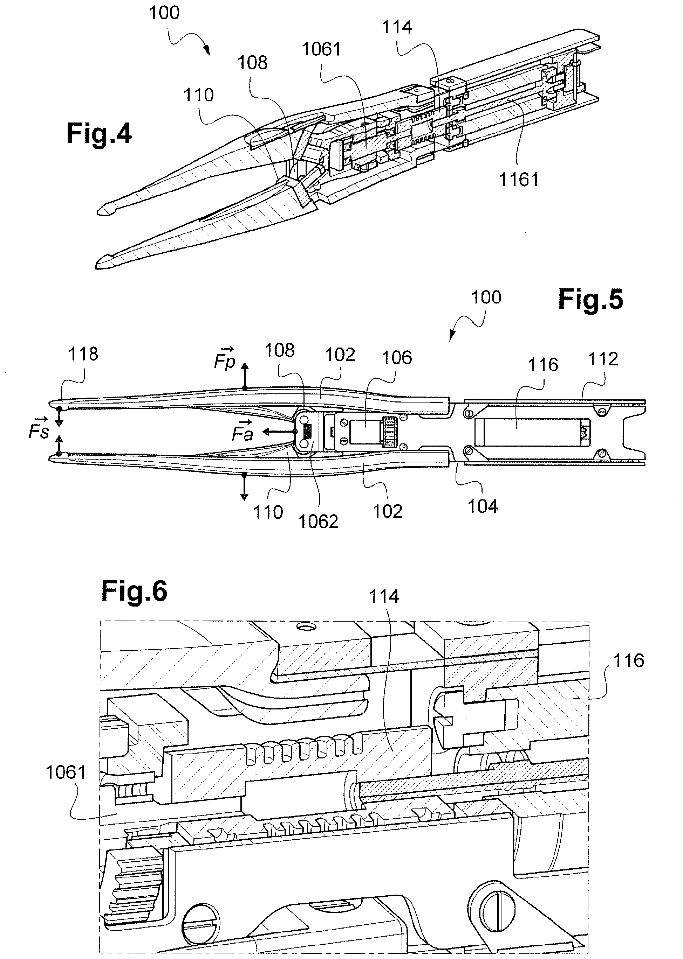

Il est maintenant fait références aux figures 3 à 7.

La pince mécatronique 100 présente un aspect général de pince brucelles droite. La pince 100 présente une symétrie générale par rapport à un axe central longitudinal. Deux branches 102 s'étendent longitudinalement à partir d'une embase 112 selon une première direction. Chaque branche 102 comprend des extrémités libres 118. Les branches 102 sont montées sur l'embase 112 de sorte à pouvoir s'approcher l'une de l'autre par un mouvement de rotation. À cet effet, chaque branche 102 est montée en rotation sur l'embase 112. Plus précisément, chaque branche 102 est fixée à l'embase 112 par une lamelle ressort 104 respective. La pince 100 comprend donc deux lamelles ressort 104. Les lamelles ressort 104 sont interchangeables afin de modifier au besoin la raideur de la pince 100.

Le rapprochement des branches 102 l'une vers l'autre par rotation résulte en un rapprochement des extrémités libres 118 l'une vers l'autre. Ainsi, les extrémités libres 118 forment une pince apte à saisir des objets. D'une manière générale, les deux branches 102 comportent des extrémités libres 118 formant pince et sont agencées pour se rapprocher par rotation l'une de l'autre depuis une position de repos à une position active.

La pince 100 est conformée pour être prise en main par un opérateur. À cet effet la pince 100 comporte une zone de préhension 120 sur chaque branche. Pendant l'utilisation, un opérateur peut notamment apposer un doigt sur chaque zone de préhension 120 afin de manipuler la pince 100, par exemple en apposant l'index et le pouce d'une main respectivement sur l'une et l'autre des branches 102.

L'embase 112 loge un moteur 116. Le moteur peut être de type électrique à courant continu, lequel dispose d'un rotor à faible inertie grâce à un noyau ferromagnétique immobile. Dans le présent exemple de réalisation, le moteur est le modèle DCX10L EB KL 4.5V de la société *MAXON MOTOR AG.* Ce moteur met en œuvre une technologie dite « coreless » à base d'aimants permanents. Les performances énergétiques ainsi que le dimensionnement de ce type de moteur sont particulièrement adaptés pour le dispositif mécatronique de l'invention. D'une manière générale le moteur est de préférence un moteur électromagnétique pour lequel la partie mobile est constituée soit par un aimant soit par une bobine.

La pince mécatronique comprend en outre un actionneur 106. Avantageusement l'actionneur 106 est une vis-à-billes 1061. En effet, les vis-à-billes présentent de bonnes performances en termes de minimisation de frottements. Une vis-à-billes comprenant un élément de vis et un élément écrou couplés l'un à l'autre de sorte qu'un mouvement de translation de l'écrou entraîne un mouvement de rotation de la vis, et *vice versa.* Dans le présent exemple de réalisation, l'actionneur 106 est une vis-à-billes de modèle 4x1 Carry Type « ZYI » de la société *EICHENBERGER GEWINDE AG.* Les performances en termes de frottements ainsi que le dimensionnement de ce type d'actionneur sont particulièrement adaptés pour le dispositif mécatronique de l'invention. Il s'agit d'une vis-à-billes miniature présentant un angle d'hélice $\theta > 4°$. Le degré de liberté en rotation de l'écrou de la vis-à-billes est bloqué.

D'autres types d'actionneurs peuvent être utilisés. Par exemple, des actionneurs comprenant un ensemble de roulements à billes se déplaçant avec une trajectoire hélicoïdale autour d'une tige lisse peuvent être utilisés [à titre d'exemple : *Roh'lix® Linear Actuators* de la société *ZERO-MAX, INC*]. Toutefois, aujourd'hui ces types d'actionneurs sont généralement encombrants.

L'actionneur 106 est agencé pour engager un mouvement de translation dans les deux sens de l'axe longitudinal central. En d'autres termes, l'actionneur 106 est apte à engager un mouvement de translation dans les deux sens de la première direction : il s'agit d'une poussée ou d'une traction. Ceci est réalisé au moyen de la vis-à-billes. En effet, comme mentionné plus haut, la rotation de la vis a pour conséquence un mouvement de translation de l'élément écrou de la vis-à-billes.

Le moteur 116 comprend un arbre moteur 1161. L'arbre moteur 1161 est entraîné en rotation. L'arbre moteur 1161 est relié à un organe de couplage flexible 114. L'organe de couplage flexible 114 est par ailleurs relié à l'actionneur 106 et plus précisément à l'élément vis de la vis-à-billes 1061. Le moteur 116 entraîne ainsi en rotation la vis-à-billes 1061 de l'actionneur 106 par le biais de l'organe de couplage flexible 114. L'organe de couplage flexible 114 monté entre le moteur 116 et l'actionneur 106 permet de palier des éventuels écarts d'axe entre l'arbre moteur 1161 et la vis-à-billes 1061.

L'entrainement en rotation de la vis-à-billes a pour conséquence un mouvement de translation de l'élément écrou de la vis 1061. À cet effet une tête 1062 est prévue sur l'actionneur 106. La tête 1062 peut ainsi exercer des forces sur la pince mécatronique 100 et tout particulièrement sur les branches 102.

Pour cela le mouvement de translation de l'actionneur 106, et précisément de la tête 1062 doit être transformé en mouvement de rotation des branches 102. À cet effet, la pince 100 comprend une ou plusieurs cames. Plus généralement, l'actionneur 106 possède au moins une première surface de contact agencée pour former came avec au moins une deuxième surface de contact respective située sur chaque branche 102.

D'une manière plus générale, lors de la fermeture de la pince par un opérateur (rapprochement des branches), la translation de l'élément écrou (tête 1062) de la vis-à-billes est convertie en rotation de l'élément vis (vis 1061) de la vis-à-billes : l'impédance de ce mouvement est alors modulée par le moteur 106. Bien évidemment, la rotation de l'élément vis peut aussi piloter la translation de l'élément écrou.

L'actionneur 106 peut donc être à l'origine d'une force à l'encontre du rapprochement des branches 102 et à l'origine d'une force en assistance du rapprochement des branches 102. Une force à l'encontre du rapprochement se traduit en pratique par un ressenti de résistance pour l'opérateur lors du rapprochement des branches (et de ce fait des extrémités libres 118) l'une vers l'autre. Inversement, une force en assistance du rapprochement se traduit en pratique par un ressenti d'aide pour l'opérateur lors du rapprochement des branches (et de ce fait des extrémités libres 118) l'une vers l'autre.

De manière très générale, l'effort exercé de la vis 1061 sur la tête 1062 est augmenté avant d'être transmis aux branches 102. On notera toutefois qu'il ne s'agit pas de même type d'effort.

Dans un mode de réalisation, l'actionneur 106 comprend des galets 108 formant la première surface. Dans ce mode de réalisation, chaque branche 102 comprend des épaulements 110 faisant saillies vers la branche opposée. Les épaulements présentent des plans inclinés compris entre environ 35° et 75°. La coopération entre galet et épaulement permet de transformer le mouvement de translation de l'actionneur 106 en un mouvement de rotation des branches 102. L'agencement de l'ensemble galet/épaulement est un organe formant came.

Dans le présent mode de réalisation, chaque branche 102 comporte deux épaulements 110. Les épaulements 110 sont disposés respectivement de part et d'autre de la branche, à proximité des bords de la branche. La pince mécatronique comporte donc quatre épaulements au total. Dans ce mode de réalisation, l'actionneur 106 porte quatre galets 108 chaque galet étant agencé pour venir au contact d'un épaulement 110 correspondant.

L'actionneur 106 peut exercer une force comprise entre 50 mN et 30 N. Ceci résulte en un ressenti pour l'opérateur d'une force comprise entre 10 mN et 6 N. En pratique, l'actionneur 106 peut engager une poussée ou une traction. Ainsi, l'actionneur 106 peut en toute rigueur exercer une force comprise entre -30 N et 30 N.

Pour l'efficacité de la transformation translation-actionneur/rotation-branches, la ou les premières surfaces de contact demeurent en prise avec la ou les deuxièmes surfaces de contact respectives. À cet effet, la Demanderesse a découvert non sans surprise que dans le dispositif mécatronique de l'invention des composants magnétiques permettaient d'établir un contact permanent et efficace entre la ou les premières surfaces de contact et la ou les deuxièmes surfaces de contact respectives, tout en répondant aux besoins énergétiques et aux contraintes de dimensionnements.

Dans le présent mode de réalisation, le contact permanent entre galet(s) 108 et épaulement(s) 110 est réalisé au moyen d'un premier composant magnétique et d'un deuxième composant magnétique. Le premier composant magnétique est ici constitué d'un aimant permanent 122 pris en sandwich par des noyaux ferromagnétiques 124. Le deuxième composant magnétique est ici constitué par un récepteur ferromagnétique 126. Cet agencement permet de canaliser le champ magnétique de l'aimant permanent afin de générer une puissante force de contact entre galet(s) et épaulement(s).

D'une manière générale, la précontrainte magnétique est assurée par un circuit composé d'une part par un aimant permanent sur lequel sont agencés des amplificateurs en matériau ferromagnétique à haute perméabilité et à faible saturation, et d'autre part par un récepteur constitué du même matériau ferromagnétique et jouissant d'un mouvement relatif par rapport au reste du circuit magnétique. Ainsi, liaison et le contact entre galet et épaulement (formant came bidirectionnelle) est assuré par l'action d'une précontrainte magnétique.

Le dispositif mécatronique comprend un moyen de commande permettant d'agir sur l'actionneur 106 et de le contrôler activement en temps réel. Ceci permet de produire un mouvement de rotation desdites branches qui exerce une force choisie de la pince dans sa position active. Le moyen de commande peut comprendre une base de données de forces prédéfinies enregistrée sur une mémoire de stockage. Ainsi, la force choisie appliqué lors de l'utilisation de la pince peut être sélectionnée parmi ces forces prédéfinies.

En utilisation, la pince mécatronique est agencée pour réaliser activement ou automatiquement des efforts de préhensions $F_p$, des efforts de saisies $F_s$ et des efforts sur l'actionneur $F_a$. Les efforts sont résumés dans le tableau 1. Selon les efforts agissant sur la pince 100 de l'invention, quatre cas de fonctionnements peuvent être mis en œuvre.

**Tableau 1 : Différents cas de fonctionnement de la pince mécatronique 100.**

|  | 1er Cas | 2ème Cas | 3ème Cas | 4ème Cas |
|---|---|---|---|---|
| $F_p$ Effort de préhension | $\neq 0$ | $=0$ | $\neq 0$ | $\neq 0$ |
| $F_s$ Effort de saisie | $=0$ | $\neq 0$ | $\neq 0$ | $\neq 0$ |
| $F_a$ Effort sur l'actionneur | $\neq 0$ | $\neq 0$ | $\neq 0$ | $=0$ |

1er Cas : L'opérateur ressent un effort s'opposant à la fermeture de la pince sans qu'aucun objet physique ne soit manipulé. Une application se trouve notamment dans la simulation de saisie d'objets ou dans la saisie virtuelle d'objets.

2ème Cas : L'opérateur peut saisir un objet sans avoir à exercer le moindre effort de fermeture (rapprochement des branches 102) avec ses doigts. Une application se trouve notamment dans le contrôle actif de la saisie d'objets fragiles.

3ème Cas : Les efforts de saisie et de préhension sont non-nuls. Une application se trouve notamment dans la saisie contrôlée en temps réel d'objets. Il s'agit du cas de fonctionnement le plus général. En effet, les autres cas ne sont que des cas particuliers du présent 3ème cas, à savoir lorsqu'un ou plusieurs des efforts en jeu sont nuls.

4ème Cas : Les efforts sur l'actionneur sont nuls. Le système actif est désactivé. Dans ces conditions, la pince 100 peut-être employée en tant que pince classique de type brucelles.

Le découplage des efforts $F_p$ et $F_s$ est traduit par l'existence d'une fonction $f$ définie telle que :

$$f \text{ (efforts } \mathbf{F_p}, \mathbf{F_s}, \mathbf{F_a}) = 0$$

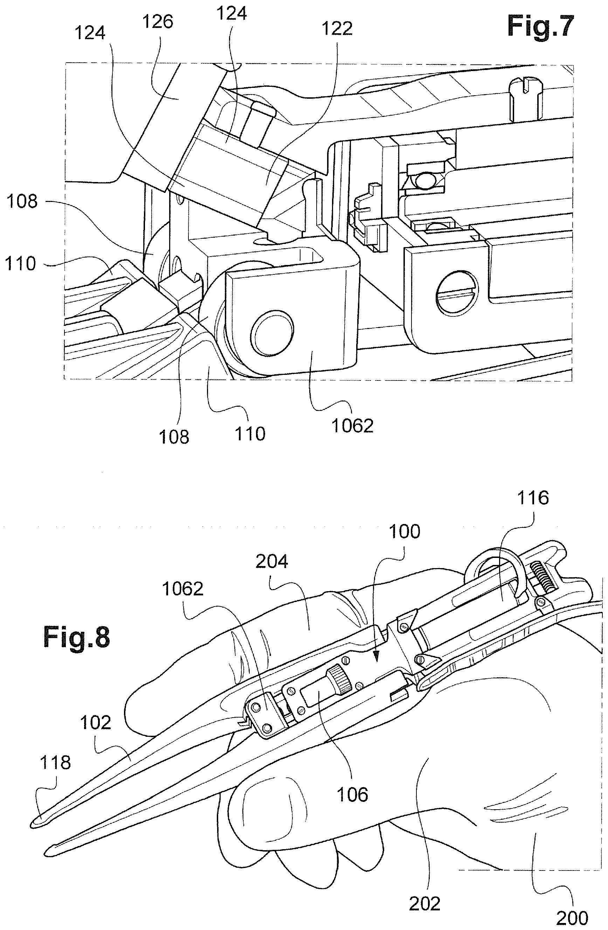

La figure 8 montre une photographie d'un dispositif mécatronique selon l'invention 100. Les branches 102 sont prises en main par un opérateur. La photographie montre une main 200. La photographie montre un premier doigt 204 (index) apposée sur une première branche 102 et un deuxième doigt 206 (pouce) apposé sur l'autre branche 102. Le moteur 116 entraîne l'actionneur 106. La tête 1062 engage ainsi un mouvement de translation qui agit directement sur l'écartement et le rapprochement des branches 102 et de ce fait respectivement sur l'ouverture et la fermeture de la pince formée par les extrémités libres 118.

L'ensemble de la chaîne cinématique, ENT., CONV., TRANS. et MANIP. composée par les différents éléments de la pince mécatronique est hautement réversible de sorte qu'aucune influence parasite (tels que les frottements ou l'inertie des pièces mécaniques mobiles) ne vienne perturber l'effort de préhension souhaitant être transmis aux doigts de l'opérateur (ou encore l'effort de saisie voulant être appliqué à un objet). Un avantage est que les éléments constituant la chaîne cinématique possèdent une grande rigidité spécifique (rapport de la raideur sur la masse) dans la direction du mouvement.

Par ailleurs, le dispositif mécatronique peut embarquer des capteurs mesurant la force perçue par les doigts de l'utilisateur, et/ou des capteurs de positions mesurant l'ouverture des branches de la pince, et/ou des capteurs permettant de déduire des coordonnées dans l'espace (par mesure des 6 degrés de liberté de position de la pince dans l'espace).

Le faible encombrement du dispositif de l'invention, sa légèreté (<40 g) et son ergonomie en font une pince mécatronique portable offrant une bonne prise en main et un confort d'utilisation supérieur aux solutions de l'état de la technique. De plus, la consommation énergétique du dispositif de l'invention est suffisamment faible pour autoriser une alimentation par une batterie.

En résumé, l'invention dispose d'un système cinématique réversible miniature qui est compatible à une implémentation sur les pinces de petites tailles telles que les brucelles. Le système cinématique est notamment composé d'un moteur à faible inertie, d'une vis-à-billes à haut rendement et d'une ensemble formant came à précontrainte magnétique. Des capteurs mesurant forces et déplacements joints à un système de commande peuvent compléter ce système. Le faible encombrement et la géométrie proche de celle d'une pince brucelles classique en font un appareil intuitif et confortable à utiliser pour un opérateur.

La pince mécatronique de l'invention permet de générer des efforts d'ouverture et de fermeture jusqu'à 6 N au niveau des doigts et 3 N aux extrémités des branches, et ce sur une course millimétrique d'environ 5 mm entre les doigts et 10 mm en bout de pince. La pince est de plus compatible avec des contraintes de portabilité telles que la compacité (144 mm x 15 mm x 22 mm), la masse (environ 40g) et la consommation énergétique. Des essais de saisie d'objets virtuels simulés par ordinateur ont été réalisés.

Des tests la saisie virtuelle d'objets ont été réalisés. La simulation d'objet peut être calculée par ordinateur notamment. Les opérateurs ont eu l'impression de physiquement saisir un objet en fermant les branches de la pince de l'invention. La saisie virtuelle est notamment rendue possible par un retour haptique s'opposant au mouvement de fermeture initié par l'opérateur.

Des tests de saisie active d'objets réels sans effort de la part de l'opérateur ont aussi été menés. Les opérateurs ont pu saisir des objets physiques sans exercer d'effort pour la fermeture de la pince.

De plus, divers tests de manipulations par des opérateurs ont été réalisés. Les tremblements de mains des opérateurs ont été compensés de manière efficace de façon à garantir la constance de l'effort de saisie. Ainsi la pince de l'invention permet de contrôler activement le mouvement et les efforts de pincements effectués par un opérateur. Les manipulations des objets à l'échelle submillimétrique par des opérateurs sont possibles avec la pince de l'invention. Par ailleurs, lorsque la pince est couplée à un robot des manipulations à l'échelle micrométrique sont possibles avec la pince de l'invention.

## Revendications

1. Dispositif mécatronique comprenant deux branches (102) s'étendant généralement à partir d'une embase (112) dans une première direction et conformées pour être prises conjointement en main par un opérateur, les deux branches comportant des extrémités libres (118) formant pince et agencées pour se rapprocher par rotation l'une de l'autre depuis une position de repos à une position active, un moteur (116) logé dans l'embase, un actionneur (106) monté entre les branches, et un moyen de commande dudit actionneur (106) qui est lié au moteur de façon à être entraîné en un mouvement de translation selon la première direction, l'actionneur possédant au moins une première surface de contact agencée pour former came avec au moins une deuxième surface de contact respective située sur chaque branche (102), la ou les premières surfaces de contact demeurant en prise avec la ou les deuxièmes surfaces de contact respectives, le moyen de commande de l'actionneur (106) étant agencé pour contrôler ledit mouvement de translation de l'actionneur afin de produire un mouvement de rotation desdites branches (102) qui exerce une force choisie de la pince dans sa position active, et dans lequel la force choisie est comprise entre 50 mN et 50 N et dans lequel l'actionneur (106) est lié au moteur (116) au moyen d'un organe de couplage flexible (114).

2.  Dispositif mécatronique selon la revendication 1, dans laquelle ladite force choisie est sélectionnée parmi une force à l'encontre du rapprochement par rotation desdites extrémités libres et une force en assistance du rapprochement par rotation desdites extrémités libres.

3.  Dispositif mécatronique selon l'une des revendications précédentes, dans lequel l'actionneur (106) est disposé sur un axe longitudinal et dans lequel ledit mouvement de translation de l'actionneur selon la première direction est choisi parmi un déplacement de l'actionneur sur l'axe longitudinal dans le premier sens de cet axe et un déplacement de l'actionneur sur l'axe longitudinal dans le deuxième sens de cet axe.

4.  Dispositif mécatronique selon l'une des revendications précédentes, dans lequel l'actionneur (106) comprend une vis-à-billes.

5.  Dispositif mécatronique selon l'une des revendications 1 à 4, dans lequel l'actionneur comprend un ou plusieurs galets (108) montés en rotation, chaque galet formant ladite première surface de contact, et dans lequel chaque branche comprend un ou plusieurs épaulements (110) faisant saillie vers la branche opposée, chaque épaulement formant ladite deuxième surface de contact respective.

6.  Dispositif mécatronique selon l'une des revendications 1 à 4, dans lequel l'actionneur (106) comprend un ou plusieurs plans inclinés, chaque plan incliné formant ladite première surface de contact, et dans lequel chaque branche comprend un ou plusieurs galets montés en rotation, chaque galet formant ladite deuxième surface de contact respective.

7.  Dispositif mécatronique selon l'une des revendications précédentes, comprenant en outre un premier composant magnétique à proximité de la ou des premières surfaces de contact, et un deuxième composant magnétique à proximité de la ou des deuxièmes surfaces de contact, les premier et deuxième composants magnétiques s'attirant mutuellement de façon à assurer au moins partiellement la situation de prise entre la ou les premières surfaces de contact et la ou les deuxièmes surfaces de contact respectives.

8.  Dispositif mécatronique selon la revendication 7, dans lequel les premier et deuxième composants magnétiques comprennent indépendamment l'un de l'autre un aimant permanent, un corps ferromagnétique et/ou un électro-aimant.

9.  Dispositif mécatronique selon l'une des revendications précédentes, dans lequel le moyen de commande comprend une base de données de forces prédéfinies enregistrée sur une mémoire de stockage, et dans lequel la force choisie est sélectionnée parmi ces forces prédéfinies.

10. Dispositif mécatronique selon l'une des revendications précédentes, dans lequel lesdites extrémités libres (118) des branches comprennent des embouts interchangeables.


**Patentansprüche**

1.  Mechatronische Vorrichtung, die zwei Äste (102) umfasst, die sich im Allgemeinen aus einer Basis (112) in einer ersten Richtung erstrecken, und ausgeformt sind, um gemeinsam durch einen Bediener in die Hand genommen zu werden, wobei die beiden Äste freie Enden (118) beinhalten, die eine Zange bilden, und angeordnet sind, um sich einander durch Drehen aus einer Ruheposition in eine aktive Position anzunähern, einen Motor (116), der in der Basis aufgenommen, ist, einen Aktor (106), der zwischen den Ästen montiert ist, und ein Mittel zum Steuern des Aktors (106), das derart an den Motor angeschlossen ist, um in eine Translationsbewegung in der ersten Richtung angetrieben zu werden, wobei der Aktor mindestens eine erste Kontaktoberfläche besitzt, die angeordnet ist, um einen Nocken mit mindestens einer jeweiligen zweiten Kontaktoberfläche zu bilden, die auf jedem Ast (102) gelegen ist, wobei die erste(n) Kontaktoberfläche(n) mit der oder den jeweiligen zweiten Kontaktoberflächen in Eingriff bleiben, wobei das Mittel zum Steuern des Aktors (106) angeordnet ist, um die Translationsbewegung des Aktors zu kontrollieren, um eine Drehbewegung der Äste (102) zu erzeugen, die eine ausgewählte Kraft der Zange in ihrer aktiven Position ausübt, und wobei die ausgewählte Kraft zwischen 50 mN und 50 N enthalten ist, und wobei der Aktor (106) anhand eines flexiblen Kopplungsorgans (114) an den Motor (116) angeschlossen ist.

**2.** Mechatronische Vorrichtung nach Anspruch 1, wobei die ausgewählte Kraft aus einer Kraft gegen die Annäherung durch Drehen der freien Enden und einer Kraft zur Unterstützung der Annäherung durch Drehen der freien Enden selektioniert wird.

**3.** Mechatronische Vorrichtung nach einem der vorstehenden Ansprüche, wobei der Aktor (106) auf einer Längsachse angeordnet ist, und wobei die Translationsbewegung des Aktors in der ersten Richtung aus einer Verschiebung des Aktors auf der Längsachse in der ersten Richtng dieser Achse und einer Verschiebung des Aktors auf der Längsachse in der zweiten Richtung dieser Achse ausgewählt wird.

**4.** Mechatronische Vorrichtung nach einem der vorstehenden Ansprüche, wobei der Aktor (106) eine Kugelrollspindel umfasst.

**5.** Mechatronische Vorrichtung nach einem der Ansprüche 1 bis 4, wobei der Aktor eine oder mehrere Kugelrollen (108) umfasst, die drehend montiert sind, wobei jede Kugelrolle die erste Kontaktoberfläche bildet, und wobei jeder Ast einen oder mehrere Ansätze (110) umfasst, die zum gegenüberliegenden Ast hervorstehen, wobei jeder Ansatz die jeweilige zweite Kontaktoberfläche bildet.

**6.** Mechatronische Vorrichtung nach einem der Ansprüche 1 bis 4, wobei der Aktor (106) eine oder mehrere geneigte Ebenen umfasst, wobei jede geneigte Ebene die erste Kontaktoberfläche bildet, und wobei jeder Ast einen oder mehrere drehend montierte Kugelrollen umfasst, wobei jede Kugelrolle die jeweilige zweite Kontaktoberfläche bildet.

**7.** Mechatronische Vorrichtung nach einem der vorstehenden Ansprüche, weiter eine erste magnetische Komponente in der Nähe der ersten Kontaktoberfläche(n), und eine zweite magnetische Komponente in der Nähe der zweiten Kontaktoberfläche(n) umfassend, wobei sich die erste und zweite magnetische Komponente gegenseitig derart anziehen, um mindestens teilweise für die Eingrifflage zwischen der (den) ersten Kontaktoberfläche(n) und der (den) jeweiligen zweiten Kontaktoberfläche(n) zu sorgen.

**8.** Mechatronische Vorrichtung nach Anspruch 7, wobei die erste und zweite magnetische Komponente unabhängig voneinander einen Dauermagneten, einen ferromagnetischen Körper und/oder einen Elektromagneten umfassen.

**9.** Mechatronische Vorrichtung nach einem der vorstehenden Ansprüche, wobei das Steuerungsmittel eine Datenbank von vorbestimmten Kräften umfasst, die in einem Ablagespeicher gespeichert ist, und wobei die ausgewählte Kraft aus diesen vorbestimmten Kräften selektioniert wird.

**10.** Mechatronische Vorrichtung nach einem der vorstehenden Ansprüche, wobei die freien Enden (118) der Äste austauschbare Endstücke umfassen.

**Claims**

**1.** A mechatronic device comprising two arms (102) extending generally from a base (112) in a first direction and shaped so as to be jointly handheld by an operator, the two arms comprising free ends (118) forming a gripper and arranged to move closer together, by rotation, from a rest position to an active position, a motor (116) housed in the base, an actuator (106) mounted between the arms, and a means for controlling said actuator (106) that is connected to the motor in a such a way as to be driven in translation in the first direction, the actuator having at least one first contact surface arranged to form a cam with at least one second respective contact surface situated on each arm (102), the first contact surface or surfaces remaining engaged with the respective second contact surface or surfaces, the means for controlling the actuator (106) being arranged to control said translation movement of the actuator in order to produce a rotational movement of said arms (102), that applies a chosen force of the gripper in its active position, and wherein the chosen force is between 50 mN and 50 N and wherein the actuator (106) is connected to the motor (116) by means of a flexible coupling member (114).

**2.** The mechatronic device according to claim 1, wherein said chosen force is selected from a force opposing rotation toward one another of said free ends and a force assisting rotation toward one another of said free ends.

**3.** The mechatronic device according to one of the preceding claims, wherein the actuator (106) is disposed on a longitudinal axis and wherein said translation movement of the actuator in the first direction is chosen from a movement of the actuator on the longitudinal axis in the first direction of that axis and a movement of the actuator on the longitudinal axis in the second direction of that axis.

4. The mechatronic device according to one of the preceding claims, wherein the actuator (106) comprises a ball-screw.

5. The mechatronic device according to one of claims 1 to 4, wherein the actuator comprises one or more rotatably mounted rollers (108), each roller forming said first contact surface, and wherein each arm comprises one or more shoulders (110) projecting toward the opposite arm, each shoulder forming said respective second contact surface.

6. The mechatronic device according to one of claims 1 to 4, wherein the actuator (106) comprises one or more inclined planes, each inclined plane forming said first contact surface, and wherein each arm comprises one or more rotatably mounted rollers, each roller forming said respective second contact surface.

7. The mechatronic device according to one of the preceding claims, further comprising a first magnetic component in the vicinity of the first contact surface or surfaces and a second magnetic component in the vicinity of the second contact surface or surfaces, the first and second magnetic components attracting each other so as to bring about at least partially the situation of engagement between the first contact surface or surfaces and the respective second contact surface or surfaces.

8. The mechatronic device according to claim 7, wherein the first and second magnetic components comprise independently of each other a permanent magnet, a ferromagnetic body and/or an electromagnet.

9. The mechatronic device according to one of the preceding claims, wherein the control means comprise a predefined force database stored in a memory and wherein the chosen force is selected from those predefined forces.

10. The mechatronic device according to one of the preceding claims, wherein said free ends (118) of the arms comprise interchangeable tips.

# Fig.1

MANIP.    TRANS.    CONV.    ENT.

# Fig.2

COMMANDE EXTÉRIEURE

ALGORITHME → MOTEUR → ACTIONNEUR → BRANCHES

Force choisie

MESURE

# Fig.3

**Fig.4**

**Fig.5**

**Fig.6**

Fig.7

Fig.8

## RÉFÉRENCES CITÉES DANS LA DESCRIPTION

**Documents brevets cités dans la description**

- WO 2015158945 A
- EP 1584414 A